# EUROPEAN PATENT SPECIFICATION

(11) **EP 1 280 522 B1**
(45) Date of publication and mention of the grant of the patent: **01.12.2004**
(21) Application number: 01920625.9
(22) Date of filing: 22.03.2001
(51) Int. Cl.: A61K 31/08, A61P 9/00

(54) **COMBINATION OF CARBOXYALKYLETHERS WITH ANTIHYPERTENSIVES AND PHARMACEUTICAL USE**
KOMBINATION VON CARBOXYALKYLETHERN MIT ANTIHYPERTENSIVA UND PHARMAZEUTISCHE VERWENDUNG
COMBINAISON DE CARBOXYALKYLETHERS AVEC DES AGENTS ANTIHYPERTENSEURS ET LEUR UTILISATION

(30) Priority: 26.04.2000 US 199855 P; 20.10.2000 US 242280 P
(43) Date of publication of application: 05.02.2003
(73) Proprietor: WARNER-LAMBERT COMPANY, Morris Plains, New Jersey 07950 (US)
(72) Inventor: AUERBACH, Bruce, Pfizer Global Res. Development, Ann Arbor, MI 48105 (US); HITCHCOCK, Karen, Diane, Morris Plains, NJ 07950 (US); RYAN, Michael, Pfizer Global Res. Development, 2800 Plymouth Road, Ann Arbor, MI 48105 (US)
(74) Representative: Simpson, Alison Elizabeth Fraser
(86) International application number: PCT/US2001/009088
(87) International publication number: WO 2001/080847

(56) References cited:
- WO-A-96/30328
- WO-A-99/30704
- US-A- 5 298 497

## Description

### FIELD OF THE INVENTION

This invention concerns a combination of an antihypertensive agent, which is known to cause a reduction in blood pressure, and a carboxyalkylether, a compound which causes a rise in high-density lipoprotein (HDL) cholesterol. The combination is useful for treating vascular disorders and preventing congestive heart failure. The carboxyalkylethers also can be used alone to treat hypertension.

### BACKGROUND OF THE INVENTION

Several clinical studies have established that lowering certain forms of cholesterol in a mammal is an effective way to treat and prevent heart attacks, sudden death, and angina, both in subjects having higher than normal levels of circulating cholesterol, as well as those having normal levels of cholesterol. Lowering low-density lipoprotein (LDL), the bad form of cholesterol, is now one of the primary objectives of physicians treating patients who have, or who have a high risk of developing, cardiovascular diseases such as coronary heart disease, atherosclerosis, myocardial infarction, stroke, cerebral infarction, and even restenosis following balloon angioplasty. Many physicians are now utilizing cholesterol lowering agents purely as a prophylactic treatment in healthy subjects whose cholesterol levels are normal, thereby guarding against development of cardiovascular diseases.

The most commonly used cholesterol lowering agents are the statins, which are compounds which inhibit the enzyme 3-hydroxy-3-methylglutaryl-coenzyme A (HMG-CoA) reductase, the enzyme responsible for catalyzing the conversion of HMG-CoA to mevalonate, which is an early and rate-limiting step in the cholesterol biosynthetic pathway. Atorvastatin and simvastatin are widely used statins.

There are several forms of circulating blood cholesterol which occur naturally in mammals. Some forms are considered "bad" cholesterol, while other forms are considered "good" cholesterol and are essential for good health. The good form of cholesterol has been established to be HDL. A bad cholesterol is LDL. Another form of LDL cholesterol, the primary bad form, is a modified form of LDL called lipoprotein(a), or "Lp(a)". High levels of Lp(a) are now believed to be detrimental and can lead to cardiovascular diseases, and is one of the major risk factors leading to death from heart disease. Carboxyalkylethers have been shown to increase HDL and lower LDL and Lp(a). Typical carboxyalkylethers are described in U.S. Patent 5,648,387 incorporated herein by reference.

Other major risk factors in developing cardiovascular disease and sudden death include hypertension and angina pectoris. Hypertension is elevated blood pressure. Numerous antihypertensive agents are now known and commonly used to lower blood pressure. Such agents include calcium channel blockers, ACE inhibitors, A-II antagonists, diuretics, beta-adrenergic receptor blockers, vasodilators, and alpha-adrenergic receptor blockers. These agents also are commonly used to treat angina pectoris, which is a severe constricting pain in the chest, often radiating from the precordium to the left shoulder and down the left arm. Angina pectoris is often caused by ischemia of the heart and is usually caused by coronary artery disease.

Because vascular diseases such as coronary heart disease, stroke, and even peripheral vascular disease, remain a leading cause of death and disability throughout the world, the need continues to develop new and improved treatments, as well as agents that will actually prevent the formation of these diseases.

We have now discovered that treatment and prevention of vascular diseases can be effected with surprising results by administering a combination of an antihypertensive agent with a carboxyalkylether. The carboxyalkylethers also can be used alone as antihypertensive agents and for treating stroke.

### SUMMARY OF THE INVENTION

This invention provides a pharmaceutical composition comprised of an effective amount of an antihypertensive agent and an effective amount of a carboxyalkylether. More particularly, the invention is a combination of an antihypertensive agent with a carboxyalkylether having Formula I 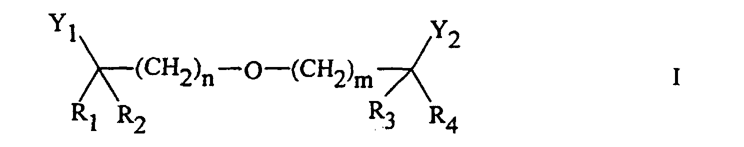 wherein
- n and m: independently are integers from 2 to 9;
- R₁, R₂, R₃, and R₄: independently are C₁-C₆ alkyl, C₁-C₆ alkenyl, C₂-C₆ alkynyl, and R₁ and R₂ together with the carbon to which they are attached, and R₃ and R₃ together with the carbon to which they are attached, can complete a carbocyclic ring having from 3 to 6 carbons;
- Y₁ and Y₂: independently are COOH, CHO, tetrazole, and COOR₅ where R₅ is C₁-C₆ alkyl, C₂-C₆ alkenyl, C₂-C₆ alkynyl;
where the alkyl, alkenyl, and alkynyl groups may be substituted with one or two groups selected from halo, hydroxy, C₁-C₆ alkoxy, and phenyl; or a pharmaceutically acceptable salt thereof.

Preferred compounds to be employed in this invention have the above formula wherein n and m are the same integer, and wherein R₁, R₂, R₃, and R₄ each are alkyl.

Further preferred are compounds wherein Y₁ and Y₂ independently are COOH or COOR₅ where R₅ is alkyl.

The most preferred compounds to be employed have the Formula II 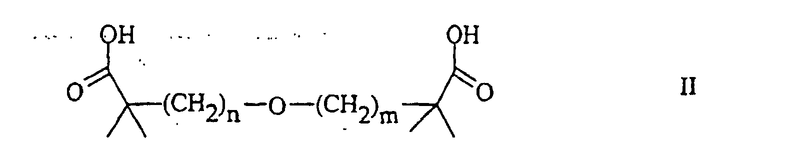 wherein n and m are each an integer selected from 2, 3, 4, or 5, ideally 4 or 5.

An especially preferred compound has the Formula III

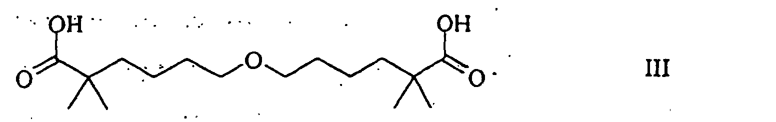

The combinations of this invention can also employ the pharmaceutically acceptable salts of the acids of Formula I. The monocalcium salt of the compound of Formula III is now known as CI-1027, and is currently being evaluated clinically for treating dyslipidemia.

The carboxyalkylethers of Formulas I, II, and III are useful for monotherapy treatment of hypertension. The use of a carboxyalkylether for the preparation of a medicament for the treatment of hypertension in a mammal is thus provided as a further embodiment of this invention.

The carboxyalkylethers can also be combined with another antihypertensive agent for combination therapy. Any antihypertensive agent can be employed in the combinations of this invention. In a preferred embodiment, the antihypertensive agent is an inhibitor of angiotensin converting enzyme (ACE inhibitor) or a calcium channel blocker.

The ACE inhibitors to be employed in the compositions of this invention are well-known in the art, and several are used routinely for clinically treating hypertension. For example, captopril and its analogs are described in US Patent 5,238,924 and 4,258,027. Enalapril, enalaprilat, and closely related analogs are described in US Patent 4,374,829, 4,472,380, and 4,264,61. Moexipril, quinapril, quinaprilat, and related analogs are described in US Patent 4,743,450 and 4,344,949. Ramipril and its analogs are described in US Patent 4,587,258 and 5,061,722. All of the foregoing patents are incorporated herein by reference for their teaching of typical ACE inhibitors which can be utilized in combination with a carboxyalkylether according to this invention. Other ACE inhibitors which can be utilized include fosinopril, fasidotril, glycopril, idrapril, imidapril, mixanpril, perindopril, spirapril, spiraprilat, temocapril, trandolapril, zofenopril, zofenoprilat, utilapril, and sampatrilat.

Another class of antihypertensive agents that can be used in the combinations of this invention are calcium channel blockers. Numerous calcium channel blockers are known, and many are routinely used clinically. Typical channel blockers include amlodipine (US 5,155,120), micardipine (US 3,985,758), nifedipine (US 3,485, 847), isradipine (US 4,466,972), felodipine (US 4,264,611), and numerous others as described in WO 99/11260. All of the references to patents and publications are incorporated herein by reference.

Another class of antihypertensive agents are called angiotensin-II receptor antagonists (A-II antagonists), and typical examples include cardesartan (US 5,196,444), eprosartan (US 5,185,351), losartan and valsartan (see US 5,399,578).

Other antihypertensive agents useful in the combinations of this invention are beta-adrenergic receptor blockers (β-blockers) such as acebutolol, alprenolol, amosulalol, arotinolol, befunolol, bevantolol, amosulalol, labetolol, buprandolol, celiprolol, cloranolol, dilevalol, epanolol, nadoxolol, perbutolol, and the like.

Similarly, alpha-adrenergic agents (α-blockers) are well-known antihypertensive agents and can be used according to this invention. Typical α-blockers include arotinolol, fenspiride, indoramin, tolazoline, trimazosin, and yohimbine. All of these α-blockers are well-known and commonly used antihypertensive agents.

Another class of antihypertensive agents are vasodilators such as bencyclane, citicoline, fasudil, ifenprodil, lomerizine, nafronyl, nimodipine, tinofedrine, and vinpocetine. All vasodilator antihypertensive agents can be used in this invention.

Diuretics also are commonly used as antihypertensive agents. These include agents such as chlorothiazide, hydrochlorothiazide, polythiazide, ambuside, butazolamide, chloraminophenamide, clofenamide, and clopamide.

All that is required to practice this invention is to combine an antihypertensive agent with a carboxyalkylether, or to otherwise use an antihypertensive agent in combination with a carboxyalkylether in the treatment of cardiovascular disorders such as atherosclerosis; hypertension, and to prevent or delay the onset of a cardiovascular event such as a heart attack.

Also provided by the invention is the use of an antihypertensive amount of a compound of Formula I for the preparation of a medicament for the treatment of hypertension in a patient in need of such treatment. A preferred use comprises using a compound of formula II or III, and most preferably by using CI-1027. The invention also provides for the use of an effective amount of a compound of formula I, and preferably a compound of formula II or III, and more preferably CI-1027, for the preparation of a medicament for the prevention of strokes in mammals.

### DETAILED DESCRIPTION OF THE INVENTION

We have discovered that combining an antihypertensive agent such as quinapril or amlodipine with a carboxyalkylether such as CI-1027 provides a surprisingly effective composition for treating and preventing vascular diseases in mammals. As noted above, the "carboxyalkylethers" as used herein are compounds such as those described in US Patent 5,648,387 incorporated herein by reference. The compounds can be the free acid, a salt form, or the tetrazolyl or aldehyde analog. These compounds can be used by themselves for treating hypertension according to this invention. They may also be used in combination with other antihypertensive agents.

The other active component of the combinations of this invention is an antihypertensive agent. Any antihypertensive agent can be used. All that is required is that such agent be effective in reducing blood pressure in a mammal.

The compositions of this invention will contain an antihypertensive agent and a carboxyalkylether in a weight ratio of about 0.01: 1 to about 1000: 1, and typically about 1:1 to about 0.5:1, and ideally about 1:1 to about 0.03:1. A typical composition, for example, will have 20 mg of quinapril hydrochloride and about 600 mg of CI-1027. All that is required is that amounts of each component are used which are effective to inhibit or reverse cardiovascular deterioration such as ventricular dilation, hypertension, and heart failure.

It will be recognized that certain of the above antihypertensive agents, for example, CI-1027, quinapril, and amlodipine contain either a free carboxylic acid or a free amine group as part of the chemical structure. Further, certain antihypertensive agents within the scope of this invention contain active moieties, which exist in equilibrium with various forms. Thus, this invention includes pharmaceutically acceptable salts of those carboxylic acids or amine groups. The expression "pharmaceutically acceptable salts" includes both pharmaceutically acceptable acid addition salts and pharmaceutically acceptable cationic salts. The expression "pharmaceutically acceptable cationic salts" is intended to define but is not limited to such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate), benzenesulfonic acid (besylate), and p-toluenesulfonate (tosylate) salts.

The pharmaceutically acceptable cationic salts of compounds containing free carboxylic acid groups may be readily prepared by reacting the free acid form of the compound with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine, and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), employing a solvent (e.g; ethyl acetate) from which the desired cationic salt precipitates, of can be otherwise isolated by concentration and/or addition of a non-solvent.

The pharmaceutically acceptable acid addition salts of compounds containing free amine groups may be readily prepared by reacting the free base form of the amine with the appropriate acid. When the salt is of a monobasic acid (e.g., the hydrochloride, the hydrobromide, the p-toluenesulfonate, the benzenesulfonate, the acetate), the hydrogen form of a dibasic acid (e.g., the hydrogen sulfate, the succinate), or the dihydrogen form of a tribasic acid (e.g., the dihydrogen phosphate, the citrate), at least one molar equivalent, and usually a molar excess, of the acid is employed. However, when such salts as the sulfate, the hemisuccinate, the hydrogen phosphate, or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

In addition, the carboxyalkylethers and pharmaceutically acceptable acid addition salts thereof may occur as hydrates or solvates. Further, the antihypertensive compounds of the instant invention and the pharmaceutically acceptable salts of such compounds may also occur as hydrates or solvates. Said hydrates and solvates are also within the scope of the invention.

The pharmaceutical combinations and uses of this invention are all adapted to therapeutic use as agents in the prevention and treatment of atherosclerosis, angina pectoris, and a condition characterized by the presence of both hypertension and hyperlipidemia in mammals, particularly humans. Further, since these diseases and conditions are closely related to the development of cardiac disease and adverse cardiac conditions, these combinations and methods, by virtue of their action as antiatherosclerotics, antianginals, antihypertensives, and antihyperlipidemics, are useful in the management of cardiac risk in subjects at risk of developing adverse cardiac conditions and in subjects at risk of suffering adverse cardiac events.

The terms "subject," "patient," and "mammal" are used interchangeably and mean animals such as humans, dogs, cats, horses, cattle, and sheep.

The utility of the compositions of the present invention as medical agents in the treatment of atherosclerosis and cardiac events in mammals (e.g., humans) is demonstrated by the activity of the compounds of this invention in conventional assays and in a clinical protocol such as those described below.

### EXAMPLE 1

The antihypertensive effects of the carboxyalkylether compound of Formula III (CI-1027), alone and in combination with an antihypertensive agent such as quinapril (CI-906), were evaluated in rats according to the following protocol.

Male spontaneously hypertensive rats (SHR, 15-20 weeks old, Charles River Laboratories, Wilmington, Massachusetts) were prepared with indwelling aortic catheters and radiotransmitters (Data Sciences International, Saint Paul, Minnesota). The radiotransmitters provided continuous measurement of aortic blood pressure waveform. From the aortic blood pressure waveform a Ponemah (Gould Instruments Inc, Valley View, Ohio) computer provided mean arterial blood pressure (MABP) and heart rate values continuously as one-minute averages. One-minute data was then summarized at 15-minute intervals using Excel (Microsoft Corporation, Redmond, Washington) spreadsheets. Animals were allowed food and water ad libitum. To allow for recovery following surgery, rats were not dosed for a minimum of 7 days. Day 0 predose blood pressures were obtained by averaging eight 15-minute MABP measurements made 2 hours before dosing. To obtain the peak response to treatment (Days 0, 12, 16, 19, and 22), the 15-minute MABP measurements in the interval between 2 and 4 hours post-dose were averaged.

Test compounds were given in a vehicle that contained 96% water, 3% carbomethocellulose (Sigma Chemical, Saint Louis, Missouri) and 1% tween 88 (polyoxyethylenesorbitan monooleate, Sigma Chemical). CI-1027 and quinapril monotherapy were administered at 8:30 AM daily by oral gavage in a volume of 2 mL/kg. The combination of CI-1027 and quinapril was given as a single gavage in a volume of 2 mL/kg. The experimental design is presented in Table 1 below.

The experiment was carried out over 22 days in 4 groups of animals. Group I served as a control and received 2 mL/kglday of vehicle for 22 consecutive days. Group II, used to determine the antihypertensive response to quinapril monotherapy, was given vehicle for the first 12 to 13 consecutive days. Then on Day 13 to 14 quinapril was added at a dose of 0.3 mg/kg/day for 3 to 4 consecutive days; the dose was then increased to 1 mg/kg/day for the next 3 days, and on the final 3 days, 3 mg/kg/day was administered. To determine the response to CI-1027 monotherapy, Groups III and IV received 12-day treatment with CI-1027 monotherapy at doses of 30 and 100 mg/kg/day, respectively. To determine the antihypertensive response to the combination therapy quinapril, was added to the ongoing CI-1027 treatment (Day 13-14) using the rising dose protocol described for Group II (quinapril monotherapy).

**Table 1.**

| Experimental Design | | | | |
|---|---|---|---|---|
| Group | Days | | | |
| | 0-12 | 13-16 | 17-19 | 20-22 |
| I | Vehicle mL/ kg | Vehicle mL/kg | Vehicle mL/kg | Vehicle mL/kg |
| | | | | |
| II | Vehicle mL/kg | Quinapril 0.3 mg/kg/day | Quinapril 1 mg/kg/day | Quinapril 3 mg/kg/day |
| | | | | |
| III | CI-1027 30 mg/kg/day | CI-1027 + Quinapril 0.3 mglkg/day | CI-1027 + Quinapril 1 mg/kg/day | CI-1027 + Quinapril 3 mg/kg/day |
| | | | | |
| IV | CI-1027 100 mg/kg/day | CI-1027 + Quinapril 0.3 mg/kg/day | CI-1027 + Quinapril 1 mg/kg/day | CI-1027 + Quinapril 3 mg/kg/day |

Among the 4 treatment groups, predose baseline MABP ranged between 141 and 149 mm Hg. Over the 22-days peak, MABP ranged between 140 mm Hg to 145 mm Hg in the Group I vehicle control rats indicating that vehicle alone had no effect on blood pressure. Twelve-day CI-1027 monotherapy at 30 mg/kg/day (Group III) significantly (Day 0 vs Day 12, p <0.05) reduced peak MABP down to 131. The MABP in rats treated with 100 mg/kg/day (Group IV) equaled that of the 30-mg/kg/day dose. Thus, CI-1027 monotherapy demonstrated a modest antihypertensive activity.

Quinapril monotherapy was given to Group III using a rising dose protocol of 3 days each at 0.3, 1, and 3 mg/kg/day. Using a rising dose protocol, 9 days of quinapril monotherapy lowered MABP down to 117 mm Hg (Day 22). Quinapril at 3 mg/kg/day yielded an antihypertensive response that was near maximal since the response that was only 5 mm Hg greater than noted at 0.3 mg/kg/day.

Results from Groups III and IV indicate that multiple-day quinapril treatment when added to ongoing CI-1027 treatment greatly potentates the antihypertensive response. MABP values in the combination treated rats were much lower that the corresponding quinapril monotherapy group. Rats treated for 3 days with the combination of quinapril at 1 m/kg/day and CI-1027 at 30 and 100 mg/kg/day had MABP values (Day 19) of 104 and 111 mm Hg, respectively. The difference in MABP reached statistical significance with the CI-1027 combination at 30 mg/kg/day. After 3-day treatment at the highest quinapril dose (3 mg/kg/day), the quinapril CI-1027 combination resulted in MABP values that were below 100 mm Hg (p <0.05 versus quinapril monotherapy).

The data from the foregoing experiment is shown in Table 2.

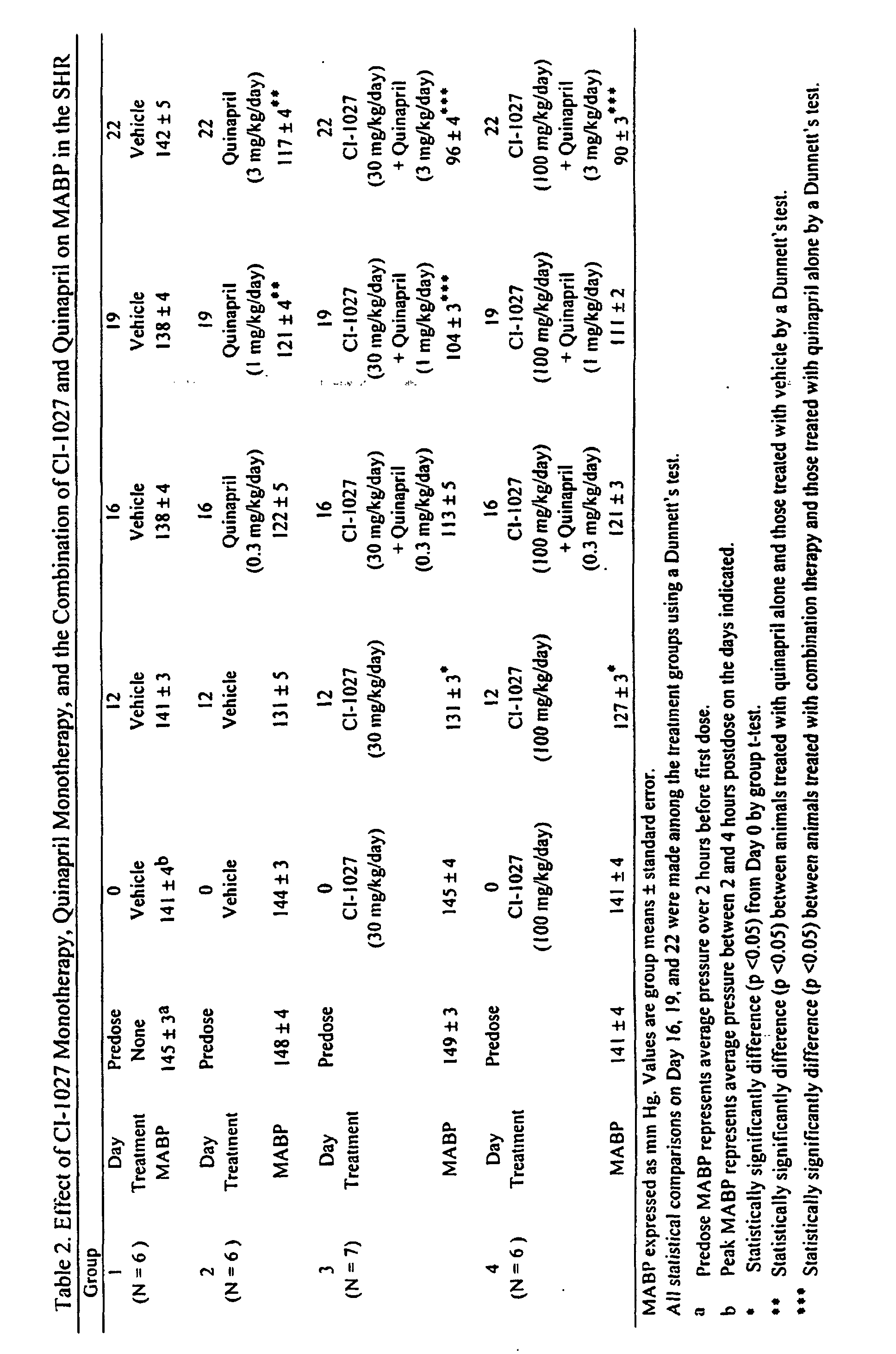

### EXAMPLE 2

The general procedure of Example 1 was followed to evaluate the antihypertensive efficacy of the carboxyalkylether CI-1027 alone and in combination with the calcium channel blocker amlodipine.

Male SHR (15-20 weeks old, Charles River Laboratories) were prepared with indwelling aortic catheters and radiotransmitters for continuous measurement of arterial blood pressure and heart rate. Animals were allowed food and water ad libitum. Before dosing all rats were given a minimum of 7 days for recovery from surgery.

The experiment was carried out over 22 days in 3 groups of SHR. Group I served as a control and received 2 mL/kg/day of vehicle (same as in Example 1) for 22 consecutive days. Group II, used to determined the antihypertensive response to amlodipine monotherapy, was given vehicle for the first 13 consecutive days. Then on Day 14, amlodipine was added at a dose of 0.3 mg/kg/day for 3 consecutive days. The dose was then increased to 1 mg/kg/day for the next 3 days, and on the final 3 days, 3 mg/kg/day was given. To determine the response to CI-1027 monotherapy, Group III received a 13-day treatment with CI-1027 monotherapy at a dose of 30 mg/kg/day. The antihypertensive response to the combination therapy was determined by the addition of amlodipine to ongoing CI-1027 treatment on Day 14. The experimental design/dosing is shown in Table 3.

**Table 3**

| Group | Days | | | |
|---|---|---|---|---|
| | 0-13 | 14-16 | 17-19 | 20-22 |
| I | Vehicle mL/kg | Vehicle mL/kg | Vehicle mL/kg | Vehicle mL/kg |
| | | | | |
| II | Vehicle mL/kg | Amlodipine 0.3 mg/kg/day | Amlodipine 1 mg/kg/day | Amlodipine 3 mg/kg/day |
| | | | | |
| III | CI-1027 30 mg/kg/day | CI-1027 + Amlodipine 0.3 mg/kg/day | CI-1027 + Amlodipine 1 mg/kg/day | CI-1027 + Amlodipine 3 mg/kg/day |

Drugs were suspended or dissolved in vehicle that contains 96% water, 3% carboxymethocellulose, and 1% tween 88. Compounds were given at 9 AM daily by oral gavage in a volume of 2 mL/kg. The combination of CI-1027 and amlodipine was given as a single gavage in a volume of 2 mL/kg.

The results of the experiment are presented in Table 4. Among the three treatment groups, predose baseline MABP ranged between 131 and 134 mm Hg. Over the 22 days, peak MABP remained constant (136-141 mm Hg) in Group I vehicle-time control rats. The first half of the experiment demonstrated that CI-1027 monotherapy lowers blood pressure in the SHR. On Day 13, MABP was 120 mm Hg in CI-1027-treated SHR, while the two vehicle groups had MABP values of 138 and 144 mm Hg.

The threshold antihypertensive dose for amlodipine monotherapy (Group II) was 1 mg/kg/day, and 3 mg/kg/day lowered MABP to 114 mm Hg. Addition of amlodipine treatment to ongoing CI-1027 treatment (Group III) resulted in an antihypertensive response that was greater than that obtained with either agent alone. For example, SHR treated for 3 days with the combination of amlodipine at 3 mg/kg/day and CI-1027 at 30 mg/kg/day (Day 22) had a MABP of only 96 mm Hg, compared to 114 mm Hg for amlodipine monotherapy. These results establish that the combination of amlodipine with CI-1027 provides for very effective control of blood pressure in hypertensive mammals.

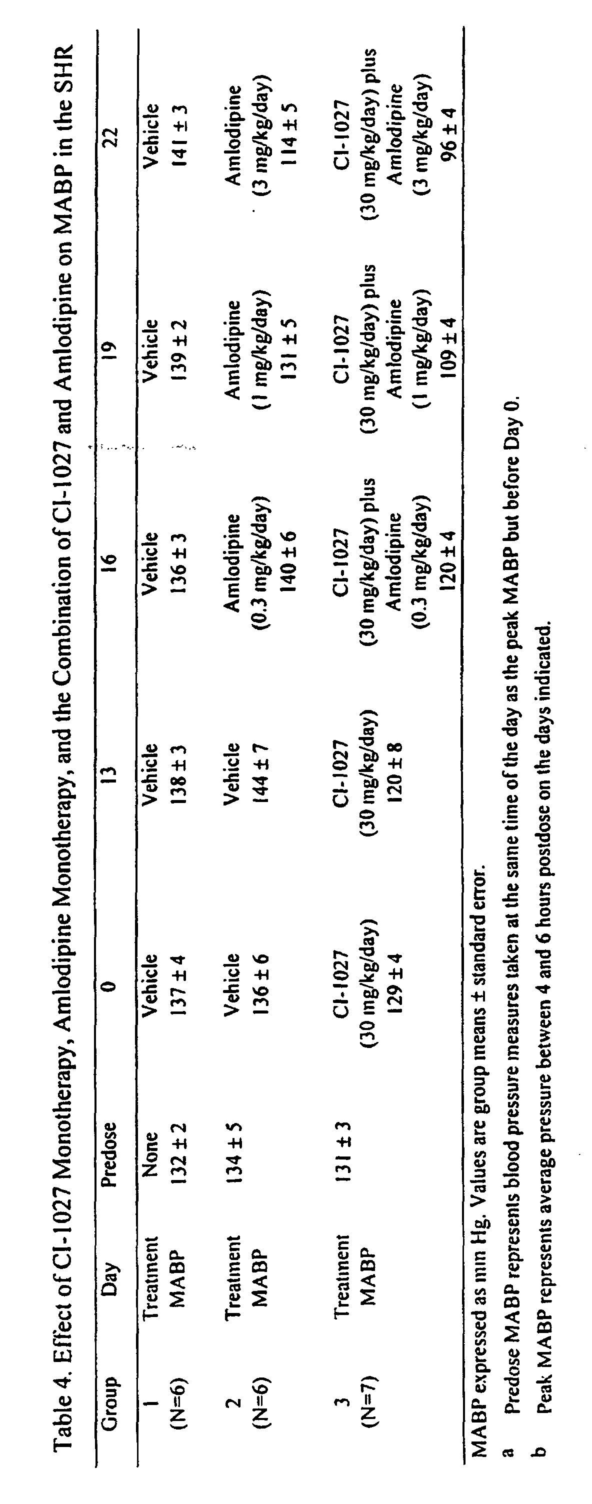

### EXAMPLE 3

### Effect of Compound of Formula III and an Antihypertensive Agent, Alone and in Combination, on the Treatment of Atherosclerosis and Cardiovascular Disease

This study is a prospective randomized evaluation of the effect of a combination of the compound of Formula III (compound III) or a pharmaceutically acceptable salt thereof and an antihypertensive agent such as amlodipine on the progression/regression of coronary and carotid artery disease and risk of heart failure. The study is used to show that a combination of the compound of Formula III or a pharmaceutically acceptable acid addition salt and an antihypertensive agent such as amlodipine besylate is effective in slowing or arresting the progression or causing regression of existing coronary artery disease (CAD) as evidenced by changes in coronary angiography or carotid ultrasound, in subjects with established disease.

This study is an angiographic documentation of coronary artery disease carried out as a double-blind, placebo-controlled trial of a minimum of about 500 subjects and preferably of about 780 to about 1200 subjects. It is especially preferred to study about 1200 subjects in this study. Subjects are admitted into the study after satisfying certain entry criteria set forth below.

*Entry criteria:* Subjects accepted for entry into this trial must satisfy certain criteria. Thus, the subject must be an adult, either male or female, aged 18 to 80 years of age in whom coronary angiography is clinically indicated. Subjects will have angiographic presence of a significant focal lesion such as 30% to 50% on subsequent evaluation by quantitative coronary angiography (QCA) in a minimum of one segment (non-PTCA, non-bypassed, or non-MI vessel) that is judged not likely to require intervention over the next 3 years. It is required that the segments undergoing analysis have not been interfered with. Since percutaneous transluminal cardiac angioplasty (PTCA) interferes with segments by the insertion of a balloon catheter, non-PTCA segments are required for analysis. It is also required that the segments to be analyzed have not suffered a thrombotic event, such as a myocardial infarct (MI). Thus, the requirement for non-MI vessels. Segments that will be analyzed include: left main, proximal, mid and distal left anterior descending, first and second diagonal branch, proximal and distal left circumflex, first or largest space obtuse marginal, proximal, mid and distal right coronary artery. Subjects will have an ejection fraction of greater than 30% determined by catheterization or radionuclide ventriculography or ECHO cardiogram at the time of the qualifying angiogram or within the previous 3 months of the acceptance of the qualifying angiogram provided no intervening event such as a thrombotic event or procedure such as PTCA has occurred.

Generally, due to the number of patients and the physical limitations of any one facility, the study is carried out at multiple sites. At entry into the study, subjects undergo quantitative coronary angiography as well as B-mode carotid artery ultrasonography and assessment of carotid arterial compliance at designated testing centers. This establishes baselines for each subject. Once admitted into the test, subjects are randomized to receive the compound of Formula III (600 mg) and placebo or an antihypertensive agent (dose is dependent upon the particular agent used; however, generally 40 mg will be used at first) and placebo or compound III (600 mg) and an antihypertensive agent (40 mg). It will be recognized by a skilled person that the free base form or other salt forms of compound III or the free base form or other salt forms of the antihypertensive agent may be used in this invention. Calculation of the dosage amount for these other forms of the antihypertensive agent and compound III is easily accomplished by performing a simple ratio relative to the molecular weights of the species involved. The amount of compound III may be varied as required. Generally, a subject will start out taking 600 mg, and the amount will be titrated down to as little as 200 mg as determined by the clinical physician. The amount of the antihypertensive agent will similarly be titrated down from 40 mg if it is determined by the physician to be in the best interests of the subject. The subjects are monitored for a 1- to 3-year period, generally 3 years being preferred. B-mode carotid ultrasound assessment of carotid artery atherosclerosis and compliance are performed at regular intervals throughout the study.

Generally, 6-month intervals are suitable. Typically this assessment is performed using B-mode ultrasound equipment. However, a person skilled in the art may use other methods of performing this assessment. Coronary angiography is performed at the conclusion of the 1- to 3-year treatment period. The baseline and posttreatment angiograms and the intervening carotid artery B-mode ultrasonograms are evaluated for new lesions or progression of existing atherosclerotic lesions. Arterial compliance measurements are assessed for changes from baseline and over the 6-month evaluation periods.

The primary objective of this study is to show that the combination of carboxyalkylether or a pharmaceutically acceptable acid addition salt and an antihypertensive agent reduces the progression of atherosclerotic lesions as measured by quantitative coronary angiography (QCA) in subjects with clinical coronary artery disease. QCA measures the opening in the lumen of the arteries measured.

The primary endpoint of the study is the change in the average mean segment diameter of the coronary artery tree. Thus, the diameter of an arterial segment is measured at various portions along the length of that segment. The average diameter of that segment is then determined. After the average segment diameter of many segments has been determined, the average of all segment averages is determined to arrive at the average mean segment diameter. The mean segment diameter of subjects taking an antihypertensive agent and compound III or a pharmaceutically acceptable acid addition salt will decline more slowly, will. be halted completely, or there will be an increase in the mean segment diameter. These results represent slowed progression of atherosclerosis, no change in the progression of atherosclerosis, and regression of atherosclerosis, respectively.

The secondary objective of this study is that the combination of carboxyalkylether or a pharmaceutically acceptable acid addition salt and an antihypertensive agent reduces the rate of progression of atherosclerosis in the carotid arteries as measured by the slope of the maximum intimal-medial thickness measurements averaged over 12 separate wall segments (Mean Max) as a function of time, more than does compound III or a pharmaceutically acceptable acid addition salt or an antihypertensive agent alone. The intimal-medial thickness of subjects taking an antihypertensive agent and compound III or a pharmaceutically acceptable salt thereof will increase more slowly, will cease to increase, or will decrease. These results represent slowed progression of atherosclerosis, halted progression of atherosclerosis, and regression of atherosclerosis, respectively. Further, these results may be used to facilitate dosage determinations.

The utility of the compounds of the present invention as medical agents in the treatment of angina pectoris in mammals (e.g., humans) is demonstrated by the activity of the compounds of this invention in conventional assays and the clinical protocol described below.

### EXAMPLE 4

### Effect of Compound of Formula III and an Antihypertensive Agent, Alone and in Combination, on the Treatment of Angina

This study is a double-blind, parallel-arm, randomized study to show the effectiveness of compound III or a pharmaceutically acceptable acid addition salt thereof and an antihypertensive agent given in combination in the treatment of symptomatic angina.

*Entry criteria:* Subjects are males or females between 18 and 80 years of age with a history of typical chest pain associated with one of the following objective evidences of cardiac ischemia: (1) stress test segment elevation of about one millimeter or more from the ECG; (2) positive treadmill stress test; (3) new wall motion abnormality on ultrasound; or (4) coronary angiogram with a significant qualifying stenosis. Generally a stenosis of about 30% to 50% is considered to be significant.

Each subject is evaluated for about 10 to 32 weeks. At least 10 weeks are generally required to complete the study. Sufficient subjects are used in this screen to ensure that about 200 to 800 subjects and preferably about 400 subjects are evaluated to complete the study. Subjects are screened for compliance with the entry criteria, set forth below, during a 4-week run-in phase. After the screening criteria are met, subjects are washed out from their current antianginal medication and stabilized on a long acting nitrate such as nitroglycerine, isosorbide-5-mononitrate or isosorbide dinitrate. The term "washed out", when used in connection with this screen, means the withdrawal of current antianginal medication so that substantially all of said medication is eliminated from the body of the subject. A period of 8 weeks is preferably allowed for both the washout period and for the establishment of the subject on stable doses of said nitrate. Subjects having one or two attacks of angina per week while on stable doses of long acting nitrate are generally permitted to skip the washout phase. After subjects are stabilized on nitrates, the subjects enter the randomization phase provided the subjects continue to have either one or two angina attacks per week. In the randomization phase, the subjects are randomly placed into one of the four arms of the study set forth below. After completing the washout phase, subjects in compliance with the entry criteria undergo 24-hour ambulatory electrocardiogram (ECG) such as Holter monitoring, exercise stress testing such as a treadmill, and evaluation of myocardial perfusion using photon emission tomography (PET) scanning to establish a baseline for each subject. When conducting a stress test, the speed of the treadmill and the gradient of the treadmill can be controlled by a technician. The speed of the treadmill and the angle of the gradient are generally increased during the test. The time intervals between each speed and gradient increase is generally determined using a modified Bruce Protocol.

After the baseline investigations have been completed, subjects are initiated on one of the following four arms of the study: (1) placebo; (2) an antihypertensive agent (about 2.5 mg to about 50 mg); (3) compound III (about 150 mg to about 900 mg); or (4) a combination of the above doses of compound III and an antihypertensive agent together. The subjects are then monitored for 2 to 24 weeks. It will be recognized by a skilled person that the free base form or other salt forms of compound III or the free base form or other salt forms of the antihypertensive agent may be used in this invention. Calculation of the dosage amount for these other forms of the antihypertensive agent and compound III is easily accomplished by performing a simple ratio relative to the molecular weights of the species involved.

After the monitoring period has ended, subjects will undergo the following investigations: (1) 24-hour ambulatory ECG, such as Holter monitoring; (2) exercise stress testing (e.g., treadmill using said modified Bruce Protocol); and (3) evaluation of myocardial perfusion. using PET scanning. Patients keep a diary of painful ischemic events and nitroglycerine consumption. It is generally desirable to have an accurate record of the number of anginal attacks suffered by the patient during the duration of the test. Since a patient generally takes nitroglycerin to ease the pain of an anginal attack, the number of times that the patient administers nitroglycerine provides a reasonably accurate record of the number of anginal attacks.

To demonstrate the effectiveness and dosage of the drug combination of this invention, the person conducting the test will evaluate the subject using the tests described. Successful treatment will yield fewer instances of ischemic events as detected by ECG, will allow the subject to exercise longer or at a higher intensity level on the treadmill or to exercise without pain on the treadmill, or will yield better perfusion or fewer perfusion defects on PET.

The utility of the compounds of the present invention as medical agents in the treatment of hypertension and hyperlipidemia in mammals (e.g., humans) suffering from a combination of hypertension and hyperlipidemia is demonstrated by the activity of the compounds of this invention in conventional assays and the clinical protocol described below.

### EXAMPLE 5

### Effects of Carboxyalkylether and an Antihypertensive Agent, Alone and in Combination, on the Treatment of Subjects Having Both Hypertension and Hyperlipidemia

This study is a double-blind, parallel-arm, randomized study to show the effectiveness of carboxyalkylether or a pharmaceutically acceptable acid addition salt thereof and an antihypertensive agent given in combination in controlling both hypertension and hyperlipidemia in subjects who have mild, moderate, or severe hypertension and hyperlipidemia.

Each subject is evaluated for 10 to 20 weeks and preferably for 14 weeks. Sufficient subjects are used in this screen to ensure that about 400 to 800 subjects are evaluated to complete the study.

*Entry criteria:* Subjects are male or female adults between 18 and 80 years of age having both hyperlipidemia and hypertension. The presence of hyperlipidemia is evidenced by evaluation of the LDL cholesterol level of the subject relative to certain positive risk factors. If the subject has no coronary heart disease (CHD) and has less than two positive risk factors, then the subject is considered to have hyperlipidemia, which requires drug therapy if the LDL of the subject is ≥190 mg/dL. If the subject has no CHD and has two or more positive risk factors, then the subject is considered to have hyperlipidemia, which requires drug therapy if the LDL of the subject is ≥160 mg/dL. If the subject has CHD, then the subject is considered to have hyperlipidemia if the LDL of the subject is ≥130 mg/dL.

Positive risk factors include: (1) male over 45, (2) female over 55 wherein said female is not undergoing hormone replacement therapy (HRT), (3) family history of premature cardiovascular disease, (4) the subject is a current smoker, (5) the subject has diabetes, (6) an HDL of less than 45 mg/dL, and (7) the subject has hypertension. An HDL of >60 mg/dL is considered a negative risk factor and will offset one of the above mentioned positive risk factors.

The presence of hypertension is evidenced by a sitting diastolic blood pressure (BP) of >90 mm Hg or sitting systolic BP of > 140 mm Hg. All blood pressures are generally determined as the average of three measurements taken 5 minutes apart.

Subjects are screened for compliance with the entry criteria set forth above. After all screening criteria are met, subjects are washed out from their current antihypertensive and lipid lowering medication and are placed on the NCEP ATP II Step 1 diet. The NCEP ATP II (adult treatment panel, 2nd revision) Step 1 diet sets forth the amount of saturated and unsaturated fat which can be consumed as a proportion of the total caloric intake. The term "washed out", where used in connection with this screen, means the withdrawal of current antihypertensive and lipid lowering medication so that substantially all of said medication is eliminated from the body of the subject. Newly diagnosed subjects generally remain untreated until the test begins. These subjects are also placed on the NCEP Step 1 diet. After the 4-week washout and diet stabilization period, subjects undergo the following baseline investigations: (1) blood pressure and (2) fasting lipid screen. The fasting lipid screen determines baseline lipid levels in the fasting state of a subject. Generally, the subject abstains from food for 12 hours, at which time lipid levels are measured.

After the baseline investigations are performed, subjects are started on one of the following: (1) a fixed dose of compound III, generally about 150 to 900 mg; (2) a fixed dose of an antihypertensive agent, generally about 2.5 mg to about 50 mg; or (3) a combination of the above doses of compound III and an antihypertensive agent together. It will be recognized by a skilled person that the free base form or other salt forms of compound III or the free base form or other salt forms of the antihypertensive agent may be used in this invention. Calculation of the dosage amount for these other forms of the antihypertensive agent and compound III is easily accomplished by performing a simple ratio relative to the molecular weights of the species involved. Subjects remain on these doses for a minimum of 6 weeks, and generally for no more than 8 weeks. The subjects return to the testing center at the conclusion of the 6 to 8 weeks so that the baseline evaluations can be repeated. The blood pressure of the subject at the conclusion of the study is compared with the blood pressure of the subject upon entry. The lipid screen measures the total cholesterol, LDL-cholesterol, HDL-cholesterol, triglycerides, apoB, very low-density lipoprotein (VLDL) and other components of the lipid profile of the subject. Improvements in the values obtained after treatment relative to pretreatment values indicate the utility of the drug combination.

The utility of the compounds of the present invention as medical agents in the management of cardiac risk in mammals (e.g., humans) at risk for an adverse cardiac event is demonstrated by the activity of the compounds of this invention in conventional assays and the clinical protocol described below.

### EXAMPLE 6

### Effects of Carboxyalkylether and an Antihypertensive Agent, Alone and in Combination, on Subjects at Risk of Future Cardiovascular Events

This study is a double-blind, parallel-arm, randomized study to demonstrate the effectiveness of carboxyalkylether or a pharmaceutically acceptable acid addition salt and an antihypertensive agent given in combination in reducing the overall calculated risk of future events in subjects who are at risk for having future cardiovascular events. This risk is calculated by using the Framingham Risk Equation. A subject is considered to be at risk of having a future cardiovascular event if that subject is more than one standard deviation above the mean as calculated by the Framingham Risk Equation. The study is used to evaluate the efficacy of a fixed combination of carboxylalkylether or a pharmaceutically acceptable acid addition salt and an antihypertensive agent in controlling cardiovascular risk by controlling both hypertension and hyperlipidemia in patients who have both mild to moderate hypertension and hyperlipidemia.

Each subject is evaluated for 10 to 20 weeks and preferably for 14 weeks. Sufficient subjects are recruited to ensure that about 400 to 800 subjects are evaluated to complete the study.

*Entry criteria:* Subjects included in the study are male or female adult subjects between 18 and 80 years of age with a baseline 5-year risk, which risk is above the median for said subject's age and sex, as defined by the Framingham Heart Study, which is an ongoing prospective study of adult men and women showing that certain risk factors can be used to predict the development of coronary heart disease. The age, sex, systolic and diastolic blood pressure, smoking habit, presence or absence of carbohydrate intolerance, presence or absence of left ventricular hypertrophy, serum cholesterol, and HDL of more than one standard deviation above the norm for the Framingham Population are all evaluated in determining whether a patient is at risk for adverse cardiac event. The values for the risk factors are inserted into the Framingham Risk Equation and calculated to determine whether a subject is at risk for a future cardiovascular event.

Subjects are screened for compliance with the entry criteria set forth above. After all screening criteria are met, patients are washed out from their current antihypertensive and lipid lowering medication and any other medication which will impact the results of the screen. The patients are then placed on the NCEP ATP II Step 1 diet, as described above. Newly diagnosed subjects generally remain untreated until the test begins. These subjects are also placed on the NCEP ATP II Step 1 diet. After the 4-week washout and diet stabilization period, subjects undergo the-following baseline investigations: (1) blood pressure; (2) fasting; (3) lipid screen; (4) glucose tolerance test; (5) ECG; and (6) cardiac ultrasound. These tests are carried out using standard procedures well-known to persons skilled in the art. The ECG and the cardiac ultrasound are generally used to measure the presence or absence of left ventricular hypertrophy.

After the baseline investigations are performed, patients will be started on one of the following: (1) a fixed dose of compound III (about 150 to 900 mg); (2) a fixed dose of quinapril (about 2.5 mg to about 50 mg); or (3) the combination of the above doses of compound III and quinapril. Patients are kept on these doses and are asked to return in 6 to 8 weeks so that the baseline evaluations can be repeated. At this time, the new values are entered into the Framingham Risk Equation to determine whether the subject has a lower, greater, or no change in the risk of future cardiovascular event.

The above assays demonstrating the effectiveness of compound III or pharmaceutically acceptable acid addition salts thereof and quinapril or pharmaceutically acceptable salts thereof in the treatment of angina pectoris, atherosclerosis, hypertension and hyperlipidemia together, and the management of cardiac risk, also provide a means whereby the activities of the compounds of this invention can be compared between themselves and with the activities of other known compounds. The results of these comparisons are useful for determining dosage levels in mammals, including humans, for the treatment of such diseases.

The following dosage amounts and other dosage amounts set forth elsewhere in this specification and in the appendant claims are for an average human subject having a weight of about 65 kg to about 70 kg. The doses to be administered are those amounts that are effective to cause a reduction in blood pressure of the mammal, which is an antihypertensive effect. The skilled practitioner will readily be able to determine the dosage amount required for a subject whose weight falls outside the 65 to 70 kg range, based upon the medical history of the subject and the presence of diseases, e.g., diabetes, in the subject. All doses set forth herein, and in the appendant claims, are daily doses.

In general, in accordance with this invention, the carboxyalkylether is generally administered in a dosage of about 150 mg to about 1000 mg. Preferably, compound III is administered in a dosage of about 600 mg. It will be recognized by a skilled person that the free base form or other salt forms of compound III may be used in this invention. Calculation of the dosage amount for these other forms of or the free base form or other salt forms of compound III is easily accomplished by performing a simple ratio relative to the molecular weights of the species involved.

In general, in accordance with this invention, the above antihypertensive agents are administered in the following dosage amounts:
Quinapril, generally about 1.0 mg to about 80 mg and preferably about 10 mg to about 40 mg;
Amlodipine besylate, generally about 2.5 mg to about 40 mg and preferably about 2.5 mg to about 10 mg;
Enalapril maleate, generally about 2.0 mg to about 50 mg and preferably about 2.5 mg to about 20 mg;
Eprosartan mesylate, generally about 100 mg to about 800 mg and preferably about 200 mg to about 400 mg;
Acebutolol hydrochloride, generally about 100 mg to about 800 mg and preferably about 200 mg to about 400 mg; and
Hydrochlorothiazide, generally about 2.5 mg to about 160 mg and preferably about 10 mg to about 80 mg.

It will be recognized by a skilled person that the free acid form or other salt forms of the above antihypertensive agents may be used in this invention. Calculation of the dosage amount for these other forms of or the free acid form or other salt forms of such antihypertensive agents is easily accomplished by performing a simple ratio relative to the molecular weights of the species involved.

The compounds of the present invention are generally administered in the form of a pharmaceutical composition comprising at least one of the compounds. of this invention together with a pharmaceutically acceptable carrier or diluent. Thus, the compounds of this invention can be administered either individually or together in any conventional oral, parenteral, or transdermal dosage form.

For oral administration, a pharmaceutical composition can take the form of solutions, suspensions, tablets, pills, capsules, powders, and the like. Tablets containing various excipients such as sodium citrate, calcium carbonate, and calcium phosphate are employed along with various disintegrants such as starch and preferably potato or tapioca starch and certain complex silicates, together with binding agents such as polyvinylpyrrolidone, sucrose, gelatin, and acacia. Additionally, lubricating agents such as magnesium stearate, sodium lauryl sulfate, and talc are often very useful for tableting purposes. Solid compositions of a similar type are also employed as fillers in soft- and hard-filled gelatin capsules; preferred materials in this connection also include lactose or milk sugar, as well as high molecular weight polyethylene glycols. When aqueous suspensions and/or elixirs are desired for oral administration, the compounds of this invention can be combined with various sweetening agents, flavoring agents, coloring agents, emulsifying agents, and/or suspending agents, as well as such diluents as water, ethanol, propylene glycol, glycerin, and various like combinations thereof.

The combinations of this invention may also be administered in a controlled release formulation such as a slow release or a fast release formulation. Such controlled release formulations of the combination of this invention may be prepared using methods well-known to those skilled in the art. The method of administration will be determined by the attendant physician or other person skilled in the art after an evaluation of the subject's condition and requirements. The generally preferred formulation of quinapril is Accupril® (quinapril, Warner-Lambert Company) as described in US Patent 4,743,450 incorporated herein by reference. A preferred calcium channel blocker is amlodipine, preferably Norvasc® (Pfizer, Inc.).

For purposes of parenteral administration. solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous, and intraperitoneal injection purposes. In this connection, the sterile aqueous media employed are all readily obtainable by standard techniques well-known to those skilled in the art.

Methods of preparing various pharmaceutical compositions with a certain amount of active ingredient are known, or will be apparent in light of this disclosure, to those skilled in this art. For examples, see *Remington's Pharmaceutical Sciences,* Mack Publishing Company, Easter, Pennsylvania., 15th Edition (1975).

Pharmaceutical compositions according to the invention may contain 0.1 % to 95% of the compound(s) of this invention, preferably 1% to 70%. In any event, the composition or formulation to be administered will contain a quantity of a compound(s) according to the invention in an amount effective to treat the condition or disease of the subject being treated.

Since the present invention relates to the treatment of diseases and conditions with a combination of active ingredients which may be administered separately, the invention also relates to combining separate pharmaceutical compositions in kit form. The kit includes two separate pharmaceutical compositions: a carboxyalkylether or a pharmaceutically acceptable acid addition salt thereof and an antihypertensive agent or a pharmaceutically acceptable salt thereof. The kit includes container means for containing the separate compositions such as a divided bottle or a divided foil packet; however, the separate compositions may also be contained within a single, undivided container. Typically, the kit includes directions for the administration of the separate components to achieve synergistic results. The kit form is particularly advantageous when the separate components are preferably administered in different dosage forms (e.g., oral and parenteral), are administered at different dosage intervals, or when titration of the individual components of the combination is desired by the prescribing physician.

It should be understood that the invention is not limited to the particular embodiments described herein, but that various changes and modifications may be made without departing from the spirit and scope of this novel concept as defined by the following claims.

## Claims

1. A pharmaceutical composition comprising:
a. an amount of a carboxyalkylether or a pharmaceutically acceptable acid addition salt thereof:
b. an amount of an antihypertensive agent or a pharmaceutically acceptable salt thereof; and
c. a pharmaceutically acceptable carrier or diluent
wherein said antihypertensive agent is selected from a calcium channel blocker, an ACE inhibitor, an α-blocker, a β-blocker, a diuretic agent or an angiotensin-II receptor antagonist.

2. A pharmaceutical composition of Claim 1 wherein said antihypertensive agent is amlodipine or quinapril, or pharmaceutically acceptable salts thereof.

3. A pharmaceutical composition of Claim 1 or Claim 2 comprising a compound of Formula I 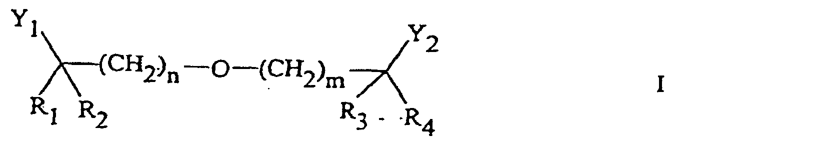 wherein n and m independently are integers from 2 to 9;
R₁, R₂, R₃ and R₄ independently are C₁-C₆ alkyl, C₁-C₆ alkenyl, C₂-C₆ alkynyl, and
R₁ and R₂ together with the carbon to which they are attached, and R₃ and R₄ together with the carbon to which they are attached, can complete a carbocyclic ring having from 3 to 6 carbons; and
Y₁ and Y₂ independently are COOH, CHO, tetrazole, and COOR₅ where R₅ is C₁-C₆ alkyl, C₁-C₆ alkenyl or C₂-C₆ alkynyl
where the alkyl, alkenyl, and alkynyl groups may be substituted with one or two groups selected from halo, hydroxy, C₁-C₆ alkoxy and phenyl; or a pharmaceutically acceptable salt thereof.

4. A pharmaceutical composition of Claim 3 comprising quinapril hydrochloride and 6,6'-oxybis(2,2-dimethylhexanoic acid) monocalcium.

5. A first pharmaceutical composition for use with a second pharmaceutical composition for achieving an antihypertensive and/or cardiovascular effect in a mammal suffering from hypertension or at risk for a cardiovascular disease, which effects are greater than the sum of the antihypertensive effects achieved by administering said first and second pharmaceutical compositions separately, and which second pharmaceutical composition comprises an amount of a carboxyalkylether or a pharmaceutically acceptable salt thereof and a pharmaceutically acceptable carrier or diluent, said first pharmaceutical composition comprising an amount of an antihypertensive agent or a pharmaceutically acceptable salt thereof and a pharmaceutically acceptable carrier or diluent
wherein said antihypertensive agent is selected from a calcium channel blocker, an ACE inhibitor, an α-blocker, a β-blocker, a diuretic agent or an angiotensin-II receptor antagonist.

6. A composition of Claim 5 wherein said antihypertensive agent is amlodipine or quinapril.

7. A composition of Claim 6 wherein said second pharmaceutical composition comprises 6,6'-oxybis(2,2-dimethylhexanoic acid) monocalcium.

8. A first pharmaceutical composition for use with a second pharmaceutical composition for achieving an antihypertensive effect in a mammal suffering from hypertension, which effect are greater than the sum of the antihypertensive effects achieved by administering said first and second pharmaceutical compositions separately and which second pharmaceutical composition comprises an amount of an antihypertensive agent or a pharmaceutically acceptable salt thereof and a pharmaceutically acceptable carrier or diluent, said first pharmaceutical composition comprising an amount of a compound of Formula II 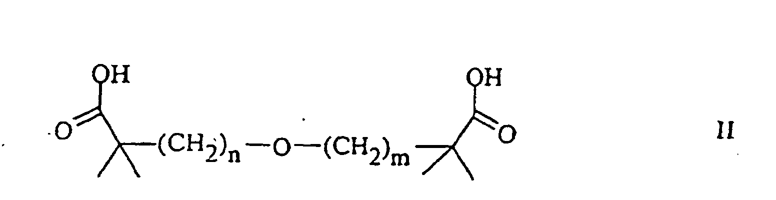 wherein n and m are each an integer selected from 2, 3, 4, or 5, or a pharmaceutically acceptable salt thereof and a pharmaceutically acceptable carrier or diluent; and wherein said antihypertensive agent is selected from a calcium channel blocker, an ACE inhibitor, an α-blocker, a β-blocker, a diuretic agent or an angiotensin-II receptor antagonist.

9. A composition of Claim 8 wherein said antihypertensive agent is amlodipine or quinapril.

10. A composition of Claim 9 comprising 6,6'-oxybis(2,2-dimethylhexanoic acid) monocalcium.

11. A first pharmaceutical composition for use with a second pharmaceutical composition for managing cardiac risk in a mammal at risk of suffering an adverse cardiac event, which effect is greater than the sum of the cardiac risk management effects achieved by administering said first and second pharmaceutical compositions separately, and which second pharmaceutical composition comprises an amount of an antihypertensive agent or a pharmaceutically acceptable salt thereof and a pharmaceutically acceptable carrier or diluent, said first pharmaceutical composition comprising an amount of a carboxyalkylether of a pharmaceutically acceptable acid addition salt thereof and a pharmaceutically acceptable carrier or diluent
wherein said antihypertensive agent is selected from a calcium channel blocker, an ACE inhibitor, an α-blocker, a β-blocker, a diuretic agent or an angiotensin-II receptor antagonist.

12. A composition of Claim 11 wherein said antihypertensive agent is amlodipine or quinapril.

13. A composition of Claim 12 comprising 6,6'-oxybis(2,2-dimethylhexanoic acid) monocalcium.

14. A kit for achieving a therapeutic effect in a mammal comprising:
a. an amount of a carboxyalkylether or a pharmaceutically acceptable acid addition salt thereof and a pharmaceutically acceptable carrier or diluent in a first unit dosage form;
b. an amount of an antihypertensive agent or a pharmaceutically acceptable salt thereof and a pharmaceutically acceptable carrier or diluent in a second unit dosage form; and
c. container means for containing said first and second dosage forms
wherein said antihypertensive agent is selected from a calcium channel blocker, an ACE inhibitor, an α-blocker, a β-blocker, a diuretic agent or an angiotensin-II receptor antagonist.

15. A kit of Claim 14 wherein said antihypertensive agent is amlodipine or quinapril.

16. A kit of Claim 15 comprising a carboxyalkylether of Formula I.

17. A kit of Claim 16 employing 6,6'-oxybis(2,2-dimethylhexanoic acid) monocalcium.

18. A kit of Claim 14 wherein said therapeutic effect is treatment of hypertension.

19. A kit of Claim 14 wherein said therapeutic effect is treatment of angina pectoris.

20. A kit of Claim 14 wherein said therapeutic is treatment of cardiac risk.

21. A kit of Claim 14 wherein said therapeutic effect is treatment of atherosclerosis.

22. A kit of Claim 21 wherein said treatment of atherosclerosis slows the progression of atherosclerotic plaques.

23. A kit of Claim 22 wherein said progression of atherosclerotic plaques is slowed in coronary arteries.

24. A kit of Claim 22 wherein said progression of atherosclerotic plaques is slowed in carotid arteries.

25. A kit of Claim 22 wherein said progression of atherosclerotic plaques is slowed in the peripheral arterial system.

26. A kit of Claim 21 wherein said treatment of atherosclerosis causes the regression of atherosclerotic plaques.

27. A kit of Claim 26 wherein said regression of atherosclerotic plaques occurs in coronary arteries.

28. The use of an effective amount of a composition according to Claim 1 for the preparation of a medicament for the treatment of vascular disease in a mammal.

29. The use of an antihypertensive effective amount of a compound of Formula I 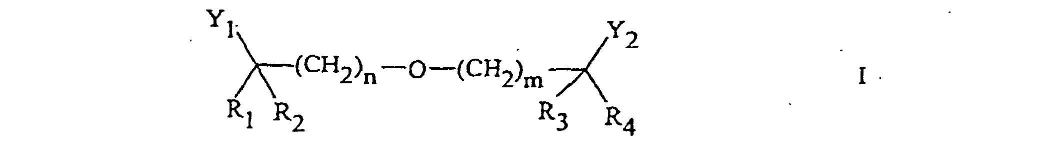 wherein n and m independently are integers from 2 to 9;
R₁, R₂, R₃ and R₄ independently are C₁-C₆ alkyl, C₁-C₆ alkenyl, C₂-C₆ alkynyl, and R₁ and R₂ together with the carbon to which they are attached, and R₃ and R₄ together with the carbon to which they are attached, can complete a carbocyclic ring having from 3 to 6 carbons;
Y₁ and Y₂ independently are COOH, CHO, tetrazole, and COOR₅, where R₅ is C₁-C₆ alkyl, C₁-C₆ alkenyl, C₂-C₆ alkynyl; and where the alkyl, alkenyl, and alkynyl groups may be substituted with one or two groups selected from halo, hydroxy, C₁-C₆ alkyoxy, and phenyl;
or a pharmaceutically acceptable salt thereof
for the preparation of a medicament for the treatment of hypertension in a mammal.

30. The use of an antihypertensive effective amount of a compound of Formula II or a pharmaceutically acceptable salt thereof 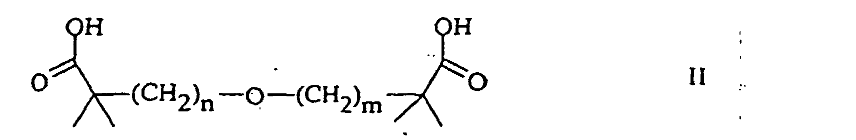 wherein n and m independently are integers from 2 to 9
for the preparation of a medicament for the treatment of hypertension in a mammal.

31. The use of an antihypertensive effective amount of a compound of Formula III 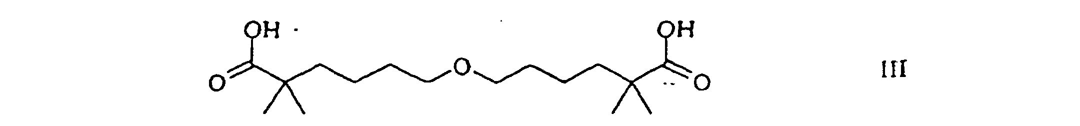 or a pharmaceutically acceptable salt thereof
for the preparation of a medicament for the treatment of hypertension in a mammal.

32. The use of an antihypertensive effective amount of 6,6'-oxybis(2,2-dimethylhexanoic acid) monocalcium for the preparation of a medicament for the treatment of hypertension in a mammal.

## Patentansprüche

1. Pharmazeutische Zusammensetzung umfassend:
a. Eine Menge eines Carboxyalkylethers oder eines pharmazeutisch verträglichen Säureadditionssalzes davon;
b. eine Menge eines Antihypertonikums oder eines pharmazeutisch verträglichen Salzes davon; und
c. einen pharmazeutisch verträglichen Träger oder ein pharmazeutisch verträgliches Verdünnungsmittel
worin genanntes Antihypertonikum ausgewählt ist aus: einem Calciumkanalblocker, einem ACE-Hemmer, einem α-Blocker, einem β-Blocker, einem Diuretikum oder einem Angiotensin-II-Rezeptor-Antagonisten.

2. Pharmazeutische Zusammensetzung nach Anspruch 1, worin genanntes Antihypertonikum Amlodipin oder Quinapril oder pharmazeutisch verträgliche Salze davon darstellt.

3. Pharmazeutische Zusammensetzung nach Anspruch 1 oder 2 umfassend eine Verbindung der Formel I 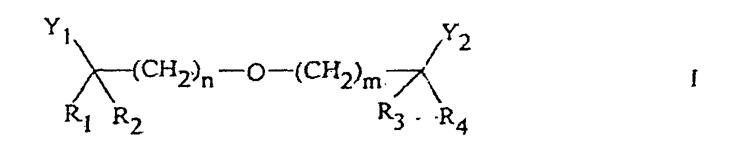 worin n und m unabhängig ganze Zahlen von 2 bis 9 darstellen; R₁, R₂, R₃ und R₄ unabhängig C₁-C₆-Alkyl, C₁-C₆-Alkenyl, C₂-C₆-Alkynyl darstellen und R₁ und R₂ zusammen mit dem Kohlenstoff, an den sie gebunden sind, und R₃ und R₄ zusammen mit dem Kohlenstoff, an den sie gebunden sind, einen carbocyclischen Ring mit von 3 bis 6 Kohlenstoffatomen vervollständigen können; und
Y₁ und Y₂ unabhängig COOH, CHO, Tetrazol und COOR₅ darstellen, worin R₅ für C₁-C₆-Alkyl, C₁-C₆-Alkenyl oder C₂-C₆-Alkynyl steht,
worin die Alkyl-, Alkenyl- und Alkynyl-Gruppen mit einer oder zwei Gruppe(n), die aus Halo, Hydroxy, C₁-C₆-Alkoxy und Phenyl ausgewählt sind, substituiert werden können; oder ein pharmazeutisch verträgliches Salz davon.

4. Pharmazeutische Zusammensetzung nach Anspruch 3 umfassend Quinapril-Hydrochlorid und 6,6'-Oxybis(2,2-dimethylhexansäure)-Monocalcium.

5. Erste pharmazeutische Zusammensetzung zur Verwendung mit einer zweiten pharmazeutischen Zusammensetzung zum Erlangen einer antihypertonen und/oder einer kardiovaskulären Wirkung in einem Säuger, der an Hypertonie leidet oder bei dem ein Risiko für eine kardiovaskuläre Erkrankung besteht, welche Wirkungen größer als die Summe der antihypertonen Wirkungen sind, die durch Verabreichung genannter erster und zweiter pharmazeutischer Zusammensetzungen getrennt erreicht werden und welche zweite pharmazeutische Zusammensetzung eine Menge eines Carboxyalkylethers oder eines pharmazeutisch verträglichen Salzes davon und einen pharmazeutisch verträglichen Träger oder ein pharmazeutisch verträgliches Verdünnungsmittel umfasst, wobei genannte erste pharmazeutische Zusammensetzung eine Menge eines Antihypertonikums oder eines pharmazeutisch verträglichen Salzes davon und einen pharmazeutisch verträglichen Träger oder ein pharmazeutisch verträgliches Verdünnungsmittel umfasst, worin genanntes Antihypertonikum aus einem Calciumkanalblocker, einem ACE-Hemmer, einem α-Blocker, einem β-Blocker, einem Diuretikum oder einem Angiotensin-II-Rezeptor-Antagonisten ausgewählt ist.

6. Zusammensetzung nach Anspruch 5, worin genanntes Antihypertonikum Amlodipin oder Quinapril darstellt.

7. Zusammensetzung nach Anspruch 6, worin genannte zweite pharmazeutische Zusammensetzung 6,6'-Oxybis(2,2-dimethylhexansäure)-Monocalcium umfasst.

8. Erste pharmazeutische Zusammensetzung zur Verwendung mit einer zweiten pharmazeutischen Zusammensetzung zum Erlangen einer antihypertonen Wirkung in einem Säuger, der an Hypertonie leidet, welche Wirkungen größer als die Summe der antihypertonen Wirkungen sind, die durch Verabreichung genannter erster und zweiter pharmazeutischer Zusammensetzungen getrennt erreicht werden und welche zweite pharmazeutische Zusammensetzung eine Menge eines Antihypertonikums oder eines pharmazeutisch verträglichen Salzes davon und einen pharmazeutisch verträglichen Träger oder ein pharmazeutisch verträgliches Verdünnungsmittel umfasst, wobei genannte erste pharmazeutische Zusammensetzung eine Menge einer Verbindung der Formel II umfasst 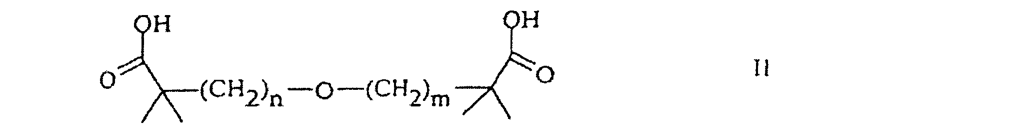 worin n und m jeweils eine ganze Zahl darstellen, die aus 2, 3, 4 oder 5, oder einem pharmazeutisch verträglichen Salz davon und einem pharmazeutisch verträglichen Träger oder ein pharmazeutisch verträgliches Verdünnungsmittel ausgewählt ist; und worin genanntes Antihypertonikum aus einem Calciumkanalblocker, einem ACE-Hemmer, einem α-Blocker, einem β-Blocker, einem Diuretikum oder einem Angiotensin-II-Rezeptor-Antagonisten ausgewählt ist.

9. Zusammensetzung nach Anspruch 8, worin genanntes Antihypertonikum Amlodipin oder Quinapril darstellt.

10. Zusammensetzung nach Anspruch 9 umfassend 6,6'-Oxybis(2,2-dimethylhexansäure)-Monocalcium.

11. Erste pharmazeutische Zusammensetzung zur Verwendung mit einer zweiten pharmazeutischen Zusammensetzung zur Behandlung des kardialen Risikos in einem Säuger, bei dem ein Risiko für das Erleiden eines unerwünschten kardialen Ereignisses besteht, welche Wirkung größer als die Summe der Wirkungen des kardialen Risikomanagements ist, die durch Verabreichung genannter erster und zweiter pharmazeutischer Zusammensetzungen getrennt erreicht werden und welche zweite pharmazeutische Zusammensetzung eine Menge eines Antihypertonikums oder eines pharmazeutisch verträglichen Salzes davon und einen pharmazeutisch verträglichen Träger oder ein pharmazeutisch verträgliches Verdünnungsmittel umfasst, wobei genannte erste pharmazeutische Zusammensetzung eine Menge eines Carboxyalkylethers eines pharmazeutisch verträglichen Säureadditionssalzes davon und einen pharmazeutisch verträglichen Träger oder ein pharmazeutisch verträgliches Verdünnungsmittel umfasst, worin genanntes Antihypertonikum aus einem Calciumkanalblocker, einem ACE-Hemmer, einem α-Blocker, einem β-Blocker, einem Diuretikum oder einem Angiotensin-II-Rezeptor-Antagonisten ausgewählt ist.

12. Zusammensetzung nach Anspruch 11, worin genanntes Antihypertonikum Amlodipin oder Quinapril darstellt.

13. Zusammensetzung nach Anspruch 12 umfassend 6,6'-Oxybis(2,2-dimethylhexansäure)-Monocalcium.

14. Kit zum Erlangen einer therapeutischen Wirkung in einem Säuger, umfassend:
a. Eine Menge eines Carboxyalkylethers oder eines pharmazeutisch verträglichen Säureadditionssalzes davon und einen pharmazeutisch verträglichen Träger oder ein pharmazeutisch verträgliches Verdünnungsmittel in einer ersten Einheitsdosierungsform;
b. eine Menge eines Antihypertonikums oder eines pharmazeutisch verträglichen Salzes davon und einen pharmazeutisch verträglichen Träger oder ein pharmazeutisch verträgliches Verdünnungsmittel in einer zweiten Einheitsdosierungsform; und
c. Behältnismittel zum Halten der genannten ersten und zweiten Dosierungsformen,
worin genanntes Antihypertonikum aus einem Calciumkanalblocker, einem ACE-Hemmer, einem α-Blocker, einem β-Blocker, einem Diuretikum oder einem Angiotensin-II-Rezeptor-Antagonisten ausgewählt ist.

15. Kit nach Anspruch 14, worin genanntes Antihypertonikum Amlodipin oder Quinapril darstellt.

16. Kit nach Anspruch 15 umfassend einen Carboxyalkylether der Formel I.

17. Kit nach Anspruch 16, das 6,6'-Oxybis(2,2-dimethylhexansäure)-Monocalcium verwendet.

18. Kit nach Anspruch 14, worin genannte therapeutische Wirkung die Behandlung der Hypertonie darstellt.

19. Kit nach Anspruch 14, worin genannte therapeutische Wirkung die Behandlung von Angina pectoris darstellt.

20. Kit nach Anspruch 14, worin genannte therapeutische Wirkung die Behandlung des kardialen Risikos darstellt.

21. Kit nach Anspruch 14, worin genannte therapeutische Wirkung die Behandlung von Atherosklerose darstellt.

22. Kit nach Anspruch 21, worin genannte Behandlung der Atherosklerose die Progression atherosklerotischer Plaques verlangsamt.

23. Kit nach Anspruch 22, worin genannte Progression atherosklerotischer Plaques in Koronararterien verlangsamt wird.

24. Kit nach Anspruch 22, worin genannte Progression atherosklerotischer Plaques in den Karotisarterien verlangsamt wird.

25. Kit nach Anspruch 22, worin genannte Progression atherosklerotischer Plaques im peripheren arteriellen System verlangsamt wird.

26. Kit nach Anspruch 21, worin genannte Behandlung der Atherosklerose zur Regression atherosklerotischer Plaques führt.

27. Kit nach Anspruch 26, worin genannte Regression atherosklerotischer Plaques in Koronararterien auftritt.

28. Verwendung einer wirksamen Menge einer Zusammensetzung nach Anspruch 1 zur Herstellung eines Arzneimittels zur Behandlung einer Gefäßerkrankung in einem Säuger.

29. Verwendung einer antihyperton wirkenden Menge einer Verbindung der Formel I 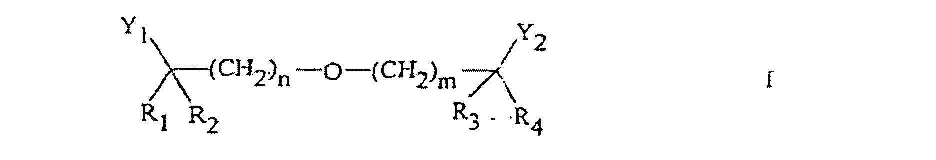 worin n und m unabhängig ganze Zahlen von 2 bis 9 darstellen; R_{1,} R₂, R₃ und R₄ unabhängig C₁-C₆-Alkyl, C₁-C₆-Alkenyl, C₂-C₆-Alkynyl darstellen, und R₁ und R₂ zusammen mit dem Kohlenstoff, an den sie gebunden sind, und R₃ und R₄ zusammen mit dem Kohlenstoff, an den sie gebunden sind, einen carbocyclischen Ring mit von 3 bis 6 Kohlenstoffatomen vervollständigen können;
Y₁ und Y₂ unabhängig COOH, CHO, Tetrazol und COOR₅ darstellen, worin R₅ C₁-C₆-Alkyl, C₁-C₆-Alkenyl, C₂-C₆-Alkynyl darstellt; und worin die Alkyl-, Alkenyl- und Alkynyl-Gruppen mit einer oder zwei Gruppe(n), die aus Halo, Hydroxy, C₁-C₆-Alkoxy und Phenyl ausgewählt sind, substituiert werden können; oder ein pharmazeutisch verträgliches Salz davon zur Herstellung eines Arzneimittels zur Behandlung der Hypertonie in einem Säuger.

30. Verwendung einer antihyperton wirkenden Menge einer Verbindung der Formel II oder eines pharmazeutisch verträglichen Salzes davon 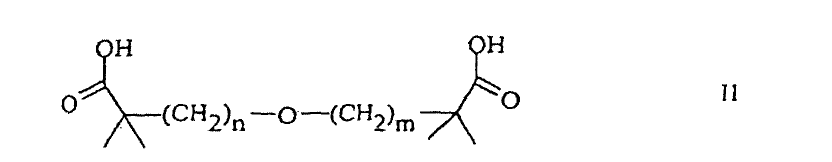 worin n und m unabhängig ganze Zahlen von 2 bis 9 zur Herstellung eines Arzneimittels zur Behandlung der Hypertonie in einem Säuger darstellen.

31. Verwendung einer antihyperton wirkenden Menge einer Verbindung der Formel III 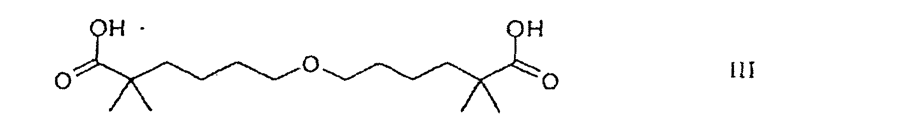 oder eines pharmazeutisch verträglichen Salzes davon zur Herstellung eines Arzneimittels zur Behandlung der Hypertonie in einem Säuger.

32. Verwendung einer antihyperton wirkenden Menge von 6,6'-Oxybis(2,2-dimethylhexansäure)-Monocalcium zur Herstellung eines Arzneimittels zur Behandlung der Hypertonie in einem Säuger.

## Revendications

1. Composition pharmaceutique comprenant :
a. une quantité de l'éther d'alkyle portant un groupement carboxy ou d'un sel d'addition d'acide pharmaceutiquement acceptable d'un tel éther ;
b. une quantité d'un agent antihypertensif ou d'un sel pharmaceutiquement acceptable d'un tel agent ; et
c. un véhicule ou un diluant pharmaceutiquement acceptable
ledit agent antihypertensif étant sélectionné parmi un antagoniste calcique, un ICE, un α-bloquant, un β-bloquant, un diurétique ou un antagoniste du récepteur à l'angiotensine-II.

2. Composition pharmaceutique selon la revendication 1, dans laquelle ledit agent antihypertensif est l'amlodipine ou le quinapril ou des sels pharmaceutiquement acceptables desdites molécules.

3. Composition pharmaceutique selon la revendication 1 ou la revendication 2, qui comprend un composé de Formule I 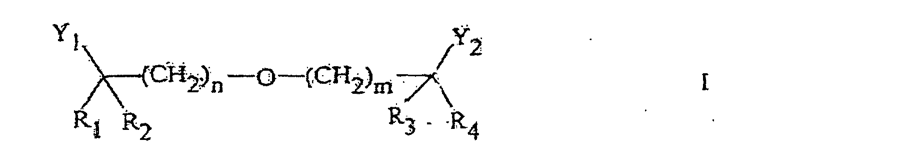 où n et m représentent des nombres entiers indépendamment sélectionnés entre 2 et 9 ;
R₁, R₂, R₃ et R₄ représentent indépendamment un alkyle en C₁-C₆, un alcényle en C₁-C₆ ou un alcynyle en C₂-C₆, et R₁ et R₂ en conjonction avec le carbone auquel ils sont rattachés et R₃ et R₄ en conjonction avec le carbone auquel ils sont rattachés peuvent former un noyau carbocyclique comportant de 3 à 6 carbones ; et
Y₁ et Y₂ représentent indépendamment COOH, CHO, un tétrazole ou COOR₅, R₅ représentant un alkyle en C₁-C₆, un alcényle en C₁-C₆ ou un alcynyle en C₂-C₆,
les radicaux alkyles, alcényles et alcynyles pouvant être substitués par un ou deux groupements sélectionnés parmi les suivants : halo, hydroxy, alkoxy en C₁-C₆ et phényle ; ou un sel pharmaceutiquement acceptable dudit composé.

4. Composition pharmaceutique selon la revendication 3, qui comprend le chlorhydrate de quinapril et un sel monocalcique de l'acide 6,6'-oxybis(2,2-diméthyl)hexanoïque.

5. Une première composition pharmaceutique à utiliser avec une seconde composition pharmaceutique pour produire un effet antihypertensif et/ou cardio-vasculaire chez un mammifère souffrant d'hypertension ou à risque de développer une maladie cardio-vasculaire, combinaison dont les effets sont supérieurs à la somme des effets antihypertensifs obtenus en administrant lesdites première et seconde compositions pharmaceutiques séparément, ladite seconde composition pharmaceutique contenant une quantité de l'éther d'un alkyle portant un groupement carboxy ou d'un sel pharmaceutiquement acceptable d'un tel éther et un véhicule ou un diluant pharmaceutiquement acceptable et ladite première composition pharmaceutique contenant une quantité d'un agent antihypertensif ou d'un sel pharmaceutiquement acceptable d'un tel agent et un véhicule ou un diluant pharmaceutiquement acceptable, ledit agent antihypertensif étant sélectionné parmi un antagoniste calcique, un ICE, un α-bloquant, un β-bloquant, un diurétique ou un antagoniste du récepteur à l'angiotensine-II.

6. Composition selon la revendication 5, dans laquelle ledit agent antihypertensif est l'amlodipine ou le quinapril.

7. Composition selon la revendication 6, dans laquelle ladite seconde composition pharmaceutique comprend un sel monocalcique de l'acide 6,6'-oxybis(2,2-diméthyl)hexanoïque.

8. Une première composition pharmaceutique à utiliser avec une seconde composition pharmaceutique pour produire un effet antihypertensif chez un mammifère souffrant d'hypertension, combinaison dont les effets sont supérieurs à la somme des effets antihypertensifs obtenus en administrant lesdites première et seconde compositions pharmaceutiques séparément, ladite seconde composition pharmaceutique contenant une quantité d'un agent antihypertensif ou d'un sel pharmaceutiquement acceptable d'un tel agent et un véhicule ou un diluant pharmaceutiquement acceptable et ladite première composition pharmaceutique contenant une quantité d'un composé de formule II 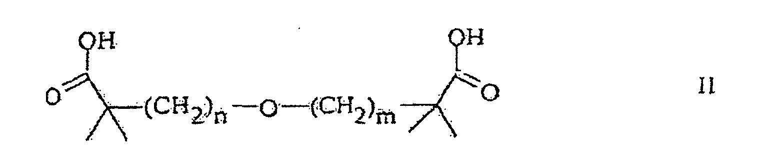 où n et m représentent chacun un nombre entier sélectionné parmi 2, 3, 4 ou 5,
ou d'un sel pharmaceutiquement acceptable dudit composé et un véhicule ou un diluant pharmaceutiquement acceptable ; ledit agent antihypertensif étant sélectionné parmi un antagoniste calcique, un ICE, un α-bloquant, un β-bloquant, un diurétique ou un antagoniste du récepteur à l'angiotensine-II.

9. Composition selon la revendication 8, dans laquelle ledit agent antihypertensif est l'amlodipine ou le quinapril.

10. Composition selon la revendication 9, qui comprend un sel monocalcique de l'acide 6,6'-oxybis(2,2-diméthyl)hexanoïque.

11. Une première composition pharmaceutique à utiliser avec une seconde composition pharmaceutique pour la prise en charge d'un risque cardiaque chez un mammifère à risque de développer un événement cardiaque fâcheux, combinaison dont les effets en termes de prise en charge d'un risque cardiaque sont supérieurs à la somme des effets obtenus en administrant lesdites première et seconde compositions pharmaceutiques séparément, ladite seconde composition pharmaceutique contenant une quantité d'un agent antihypertensif ou d'un sel pharmaceutiquement acceptable d'un tel agent et un véhicule ou un diluant pharmaceutiquement acceptable et ladite première composition pharmaceutique contenant une quantité de l'éther d'un alkyle portant un groupement carboxy ou d'un sel d'addition d'acide pharmaceutiquement acceptable d'un tel éther et un véhicule ou un diluant pharmaceutiquement acceptable, ledit agent antihypertensif étant sélectionné parmi un antagoniste calcique, un ICE, un α-bloquant, un β-bloquant, un diurétique ou un antagoniste du récepteur à l'angiotensine-II.

12. Composition selon la revendication 11, dans laquelle ledit agent antihypertensif est l'amlodipine ou le quinapril.

13. Composition selon la revendication 12, qui comprend un sel monocalcique de l'acide 6,6'-oxybis(2,2-diméthyl)hexanoïque.

14. Coffret visant à produire un effet thérapeutique chez un mammifère, qui comprend :
a. une quantité de l'éther d'un alkyle portant un groupement carboxy ou d'un sel d'addition d'acide pharmaceutiquement acceptable d'un tel éther et un véhicule ou un diluant pharmaceutiquement acceptable dans une première forme pharmaceutique unitaire ;
b. une quantité d'un agent antihypertensif ou d'un sel pharmaceutiquement acceptable d'un tel agent et un véhicule ou un diluant pharmaceutiquement acceptable dans une seconde forme pharmaceutique unitaire ; et
c. un moyen de contenir lesdites première et seconde formes pharmaceutiques
ledit agent antihypertensif étant sélectionné parmi un antagoniste calcique, un ICE, un α-bloquant, un β-bloquant, un diurétique ou un antagoniste du récepteur à l'angiotensine-II.

15. Coffret selon la revendication 14, dans lequel ledit agent antihypertensif est l'amlodipine ou le quinapril.

16. Coffret selon la revendication 15, qui comprend l'éther d'un alkyle portant un groupement carboxy répondant à la Formule I.

17. Coffret selon la revendication 16, qui utilise un sel monocalcique de l'acide 6,6'-oxybis(2,2-diméthyl)hexanoïque.

18. Coffret selon la revendication 14, pour lequel ledit effet thérapeutique est le traitement de l'hypertension.

19. Coffret selon la revendication 14, pour lequel ledit.effet thérapeutique est le traitement de l'angor.

20. Coffret selon la revendication 14, pour lequel ledit effet thérapeutique est le traitement d'un risque cardiaque.

21. Coffret selon la revendication 14, pour lequel ledit effet thérapeutique est le traitement de l'athérosclérose.

22. Coffret selon la revendication 21, avec lequel ledit traitement de l'athérosclérose ralentit l'évolution des plaques athéroscléreuses.

23. Coffret selon la revendication 22, avec lequel ladite évolution des plaques athéroscléreuses est ralentie dans les artères coronaires.

24. Coffret selon la revendication 22, avec lequel ladite évolution des plaques athéroscléreuses est ralentie dans les artères carotides.

25. Coffret selon la revendication 22, avec lequel ladite évolution des plaques athéroscléreuses est ralentie dans la circulation artérielle périphérique.

26. Coffret selon la revendication 21, avec lequel ledit traitement de l'athérosclérose cause une régression des plaques athéroscléreuses.

27. Coffret selon la revendication 26, avec lequel ladite régression des plaques athéroscléreuses a lieu dans les artères coronaires.

28. Utilisation, en une quantité efficace, d'une composition selon la revendication 1 dans la préparation d'un médicament indiqué dans le traitement d'une maladie vasculaire chez un mammifère.

29. Utilisation, en une quantité exerçant des effets antihypertensifs, d'un composé de Formule I 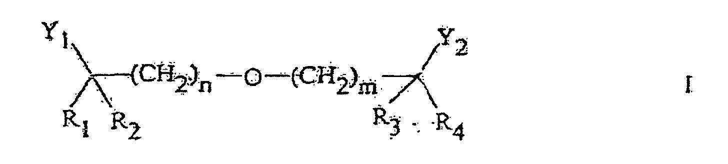 où n et m représentent des nombres entiers indépendamment sélectionnés entre 2 et 9 ;
R₁, R₂, R₃ et R₄ représentent indépendamment un alkyle en C₁-C₆, un alcényle en C₁-C₆ ou un alcynyle en C₂-C₆, et R₁ et R₂ en conjonction avec le carbone auquel ils sont rattachés et R₃ et R₄ en conjonction avec le carbone auquel ils sont rattachés peuvent former un noyau carbocyclique comportant de 3 à 6 carbones ;
Y₁ et Y₂ représentent indépendamment COOH, CHO, un tétrazole ou COOR₅, R₅ représentant un alkyle en C₁-C₆, un alcényle en C₁-C₆ ou un alcynyle en C₂-C₆ ;
et les radicaux alkyles, alcényles et alcynyles pouvant être substitués par un ou deux groupements sélectionnés parmi les suivants : halo, hydroxy, alkoxy en C₁-C₆ et phényle ; ou d'un sel pharmaceutiquement acceptable dudit composé, dans la préparation d'un médicament indiqué dans le traitement de l'hypertension chez un mammifère.

30. Utilisation, en une quantité exerçant des effets antihypertensifs, d'un composé de Formule II 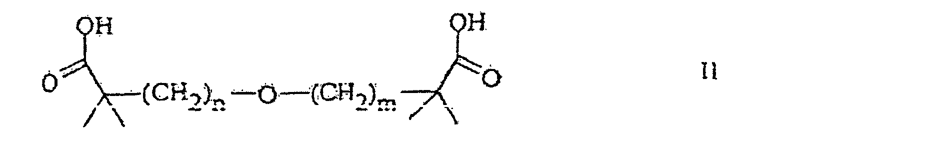 où n et m représentent des nombres entiers
indépendamment sélectionnés entre 2 et 9,
ou d'un sel pharmaceutiquement acceptable dudit composé, dans la préparation d'un médicament indiqué dans le traitement de l'hypertension chez un mammifère.

31. Utilisation, en une quantité exerçant des effets antihypertensifs, d'un composé de Formule III 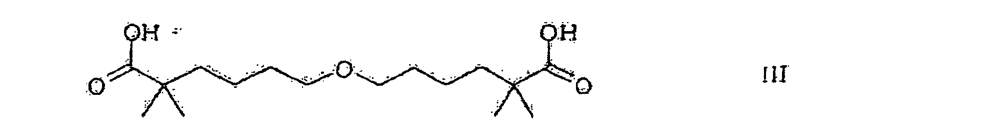 ou d'un sel pharmaceutiquement acceptable dudit composé, dans la préparation d'un médicament indiqué dans le traitement de l'hypertension chez un mammifère.

32. Utilisation, en une quantité exerçant des effets antihypertensifs, d'un sel monocalcique de l'acide 6,6'-oxybis(2,2-diméthyl)hexanoïque dans la préparation d'un médicament indiqué dans le traitement de l'hypertension chez un mammifère.
